# EUROPÄISCHE PATENTANMELDUNG

(11) **EP 0 541 108 A1**
(43) Veröffentlichungstag der Anmeldung: **12.05.1993**
(21) Anmeldenummer: 92119033.6
(22) Anmeldetag: 06.11.1992
(51) Int. Cl.: C12Q 1/00, C12Q 1/26, C12M 1/34, C12N 1/04

(54) **Arthrobacter-Nicotiana-Stamm, Aktivität des Stamms, Verfahren zur Isolierung des Stamms, Sensor mit der Aktivität und Verwendung des Stamms**

(30) Priorität: 08.11.1991 DE 4136844; 02.04.1992 DE 4211046
(71) Anmelder: Gesellschaft für biotechnologische Forschung mbH (GBF), D-38124 Braunschweig (DE)
(72) Erfinder: Wagner, Gabriele, W-3300 Braunschweig (DE); Ukeda, Hiroyuki, Dr., W-3300 Braunschweig (DE); Schmid, Rolf, Prof.Dr., W-3300 Braunschweig (DE)
(74) Vertreter: Boeters, Hans Dietrich, Dr.

(57) **Zusammenfassung**

Die Erfindung betrifft einen Arthrobacter-nicotiana-Stamm mit einer Acyl-CoA-Oxidase-Aktivität, die für C₄₋₁₂-Fettsäuren spezifisch ist, diese Aktivität, ein Verfahren zur Isolierung der Aktivität oder eines Stamms mit dieser Aktivität, einen Sensor mit der Aktiviät und dessen Verwendung.

## Beschreibung

Ranziger oder verdorbener Geruch von Milch und Milchprodukten wird durch die Freisetzung kurzkettiger C₄₋₁₂-Fettsäuren durch Milchlipase oder durch bakterielle Lipasen verursacht (1, 2). Daher ist die Entdeckung von kurzkettigen Fettsäuren bei der qualitativen Bewertung von Molkereiprodukten bei den praktizierten Methoden zum Milchsammeln und zur verlängerten Rohmilchlagerung von großer Bedeutung.

Die quantitative Bestimmung von freien Fettsäuren ist bisher durch titrimetrische Bestimmung der Gesamtsäure oder durch kolorimetrische Analysen auf Basis der Übertragung von Metallseifen aus einem Kupfer- oder Kobaltnitrat/Triethanolamin-Reagens nach der Extraktion der freien Fettsäuren in ein organisches Lösungsmittel durchgeführt worden (3, 4). Diese Methoden sind für die Kettenlänge bei Fettsäuren nicht spezifisch. Da kurzkettige Fettsäuren etwa 5 bis 6 % der Gesamtfettsäuren darstellen, haben ihre Anwesenheit oder ihr Fehlen nur einen geringen Einfluß auf den Endwert. Die Bestimmung freier Fettsäuren durch Gaschromatographie ist gleichfalls möglich (5, 6). Diese Methode gestattet es, jede Fettsäure separat zu bestimmen, jedoch hängt die quantitative Abtrennung kurzkettiger Fettsäuren zu einem hohen Grad von der Methode ab, die zur Isolierung freier Fettsäuren aus einer Probe angewendet wurde, wobei die Arbeitsweise im allgemeinen kompliziert ist. Ferner ist die enzymatische Bestimmung freier Fettsäuren vorgeschlagen worden (7, 8). Diese Methode basiert auf der Aufzeichnung von Wasserstoffperoxid, das bei zwei aufeinanderfolgenden Reaktionen durch die Enzyme Acyl-CoA-Synthetase und Acyl-CoA-Oxidase gebildet wird. Diese Methode bietet einige Vorteile hinsichtlich Selektivität und Geschwindigkeit, wobei bereits ein Sensorsystem auf Basis dieser Methode vorgeschlagen worden ist (9). Bei der Anwendung dieser Methode zur Beschreibung der Ranzigkeit von Molkereiprodukten stellt die Spezifität jedoch ein Problem dar. Das Enzym Acyl-CoA-Oxidase, das im Handel erhältlich ist, hat im allgemeinen eine hohe Spezifität gegenüber langkettigen Fettsäuren, jedoch nur eine sehr geringe Spezifität für kurzkettige Fettsäuren, insbesondere Buttersäure (7, 8).

Erfindungsgemäß wurde nun festgestellt, daß der Mikroorganismus Arthrobacter nicotiana eine Acyl-CoA-Oxidase-Aktivität besitzt, die für kurzkettige Fettsäuren hochspezifisch ist, so daß dieser Mikroorganismus erfolgreich für die Bestimmung kurzkettiger Fettsäuren verwendet werden kann.

Erfindungsgemäß wird daher ein rasches, zweckmäßiges und selektives Verfahren zur Bestimmung kurzkettiger Fettsäuren unter Einsatz des genannten Mikroorganismus nach der Fließ-Injektions-Analysen-Technik vorgeschlagen, insbesondere bei der Anwendung zur Analyse von Molkereiprodukten.

Der erfindungsgemäße Sensor umfaßt eine Mikroorganismusmembran, eine Schutzmembran und eine Sauerstoffelektrode.

Erfindungsgemäß läßt sich der Sensor zur Analyse von Molkereiprodukten einsetzen. Bei den Molkereiprodukten kann es sich um eine komplexe Mischung vieler Verbindungen handeln, wobei der Kaseingehalt in charakteristischer Weise sehr groß sein kann. Daher ist ein wichtiges Kriterium bei der Auswahl einer geeigneten Schutzmembran darin zu sehen, daß die Porengröße der Membran klein genug ist, Kaseinmoleküle daran zu hindern, mit der Mikroorganismusmembran in Berührung zu kommen. Unter diesem Gesichtspunkt kann man daher eine Dialysemembran mit einer Trenngrenze von etwa 12000 als Schutzmembran verwenden.

Die Erfindung betrifft also ein Fließ-Injektions-Analyse-System für kurzkettige Fettsäuren, bei dem eine mikrobielle Elektrode eingesetzt wird, die den Mikroorganismus Arthrobacter nicotiana, eine Dialysemembran und eine Sauerstoffelektrode aufweist. Die Antwort dieses Systems zeigt eine hohe Spezifität für kurzkettige C₄₋₁₂-Fettsäuren und eine lineare Abhängigkeit von der Konzentration von Buttersäure im Bereich von 0,11 bis 1,7 mM. Die Probenfrequenz betrug dabei etwa 20 Proben/h bei einer Fließrate von 1,0 ml/min. Die mikrobielle Elektrode ist hochselektiv und stabil. Die Erfindung eignet sich zur Aufzeichnung der Lipolyse von Rohmilch bei der Lagerung.

Eine weitere Ausführungsform der Erfindung betrifft die Verwendung eines erfindungsgemäßen Sensors zur Mastitis-Bestimmung. Es ist nämlich bei Großversuchen gezeigt worden, daß die Carboxylesterase-Aktivität von Mastitis-Milch mit dem Schweregrad der Erkrankung ansteigt und eine hochgradige Korrelation mit Mastitis-Werten besteht. Die Wirkung der Carboxylesterase ist dabei allgemein auf kurzkettige Fettsäureester beschränkt (12, 13). Diese Zusammenhänge macht sich die erfindungsgemäße Verwendung zunutze.

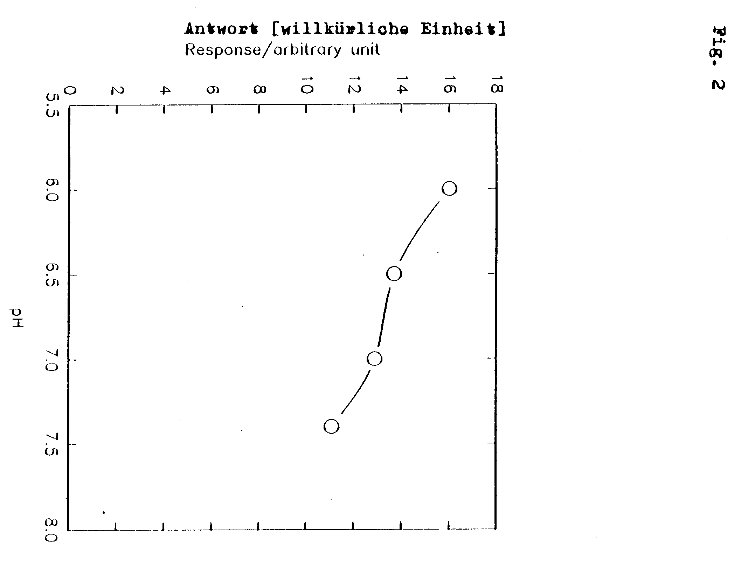

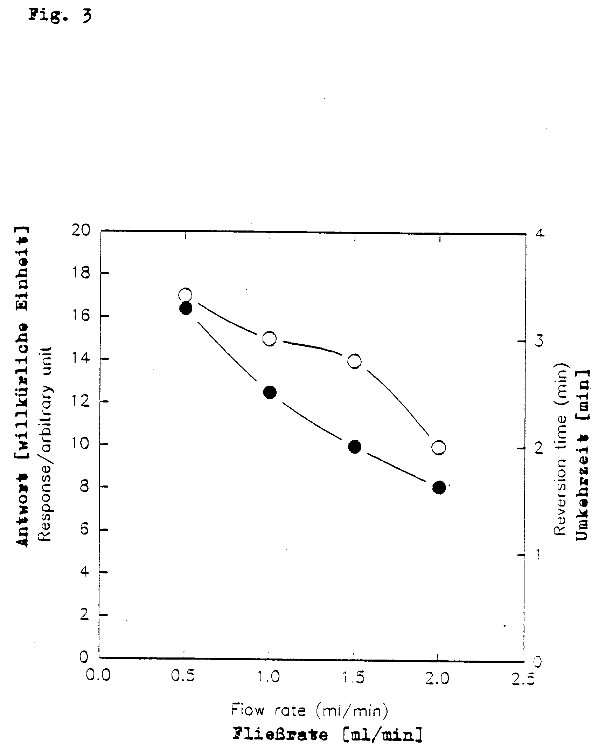

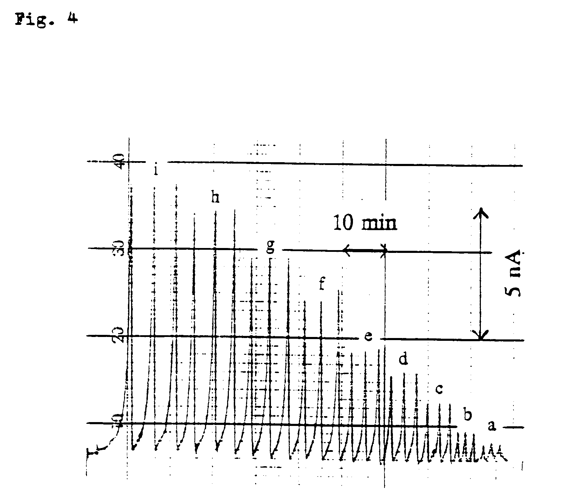

**Optimierung eines FIA-Systems**. Es wird die pH-Abhängigkeit einer mikrobiellen Elektrode im pH-Bereich von 6,0 bis 7,5 getestet (**Fig. 2**). Obgleich die Raten zu Beginn allmählich bei fallendem pH steigen, wird der Reaktionspuffer bei weiteren Versuchen bei etwa pH 7,0 gehalten, und zwar im Hinblick auf die Stabilität des eingesetzten Mikroorganismus. Wenn man die Fließrate im Bereich von 0,5 bis 2,0 ml/min ändert, fallen sowohl die Antwort als auch die Zeit für eine Grundlinienumkehr (base line reversion; 90 %) bei steigender Fließrate ab (**Fig.** **3**). Die Wahl der Fließraten stellt einen Kompromiß zwischen Sensitivität und Frequenz der Probennahme dar. Wenn beispielsweise die Strömungsrate 1,0 ml/min beträgt, ist die Antwort des Sensors im Bereich von 0,11 bis 1,7 mM-n-Buttersäure linear (**Fig. 4**), wobei die Genauigkeit bei fünf aufeinanderfolgenden Bestimmungen besser als 1,0 % ist. Die Frequenz der Probennahme beträgt etwa 20 Proben/h.

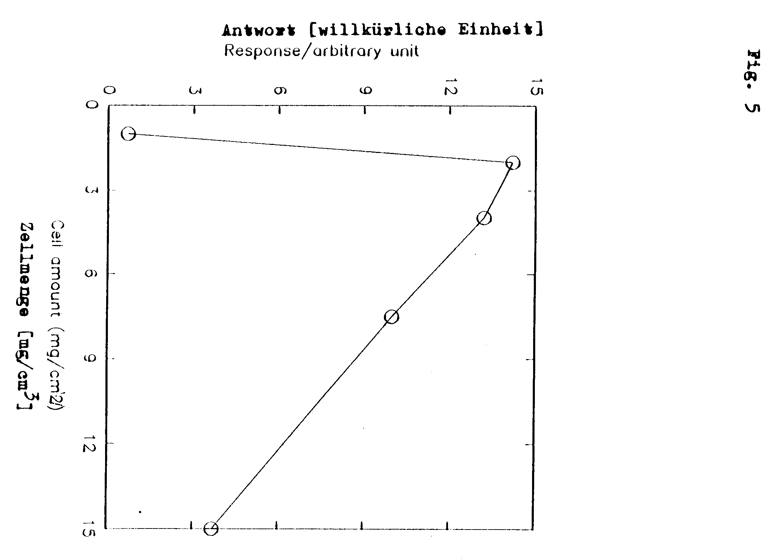

**Optimale Bedingungen zur Immobilisierung. Figur 5** zeigt den Einfluß der Zellmenge in einer PVA-Membran auf die Antwort. Die maximale Antwort wird bei Zellmengen im Bereich von 2 bis 4 mg/cm² beobachtet. Bei hohen Konzentrationen des Mikroorganismus wird die Antwort sehr klein.

**Spezifität der Antwort auf Fettsäuren**. Es ist bekannt, Standardlösungen für ein Humanblut-Muster dadurch vorzusehen, daß man freie Fettsäuren und einen Puffer mit einem Gehalt an Rinderserum-Albumin mischt (9, 11). Erfindungsgemäß wird eine Lösung dadurch vorgesehen, daß man Fettsäuren und einen Puffer mit einem Gehalt an Kasein mischt, da Kasein ein Hauptprotein in Molkereiprodukten ist. **Tabelle 1** zeigt einen Vergleich der Antwort für verschiedene Spezies von Fettsäuren in Milch. Der Sensor zeigt die hohe Spezifität für kurzkettige Fettsäuren im Bereich von Buttersäure (C_{4:0}) bis Laurinsäure (C_{12:0}). Diese Fettsäuren werden durch Milchlipase oder durch bakterielle Lipasen freigesetzt und tragen hauptsächlich zum ranzigen Geruch von Molkereiprodukten bei (1). Diese Ergebnisse legen nahe, daß der erfindungsgemäße Sensor zur Bestimmung des ranzigen Geruchs von Molkereiprodukten geeignet ist.

**Tabelle 1**

| Vergleich von Antworten auf verschiedene Fettsäuren | | |
|---|---|---|
| Fettsäure | Aufzeichnung | relativer Wert |
| Buttersäure | 4:0 | 59 |
| Capronsäure | 6:0 | 62 |
| Caprylsäure | 8:0 | 100 |
| Caprinsäure | 10:0 | 80 |
| Laurinsäure | 12:0 | 47 |
| Myristinsäure | 14:0 | 9 |
| Palmitinsäure | 16:0 | 1 |
| Stearinsäure | 18:0 | 1 |
| Oleinsäure | 18:1 | 1 |
| Linolsäure | 18:2 | 6 |

**Selektivität**. Um die Selektivität der erfindungsgemäßen mikrobiellen Elektrode zu testen, wurden die Antworten auf folgende Verbindungen bestimmt: Kasein (3 %), Rinderserum-Albumin (0,04 %), Lactose (4,6 %), Galaktose (10 mg-%), Glukose (10 mg-%), L-Glycin (3 mg-%), Cholin (30 mg-%), Creatin (3 mg-%), Harnstoff (30 mg-%), L-Lactat (3 mg-%), Citrat (0,2 %), ß-Lactoglobulin (0,5 %) und Triolein (1 %). Jede Konzentration wird auf einen für Milch üblichen Spiegel gemäß der Literatur eingestellt (1). Für diese Verbindungen wird keine Antwort beobachtet. Die erfindungsgemäße Methode basiert dabei auf der Anwendung der erfindungsgemäßen mikrobiellen Elektrode, die mit einer Dialysemembran mit kleiner Porengröße bedeckt ist.

Zusätzlich wird die Elektrode in eine Strecke mit kontinuierlichem Fluß eingesetzt, was bedeutet, daß die Verweilzeit der injizierten Probe in der Durchflußzelle, die mit der mikrobiellen Elektrode versehen ist, sehr kurz ist. Daher muß es sich bei einer Verbindung mit hoher Diffusionsrate über die Dialysemembran und hoher Assimilierbarkeit durch den immobilisierten Mikroorganismus um eine spezifische Verbindung handeln, wenn sie im System eine Antwort zeigt.

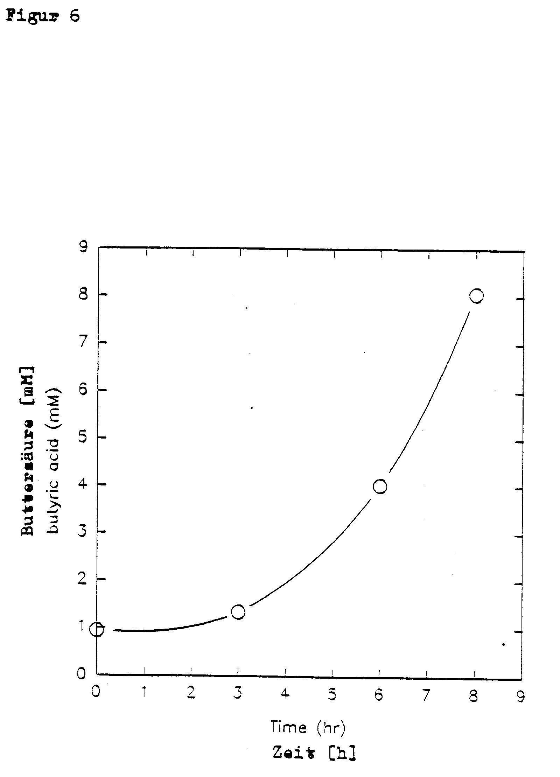

**Anwendung der mikrobiellen Elektrode**. Die erfindungsgemäße mikrobielle Elektrode wird zur Bestimmung von kurzkettigen Fettsäuren in Milch eingesetzt. Es werden vorgegebene Mengen an Buttersäure bzw. Caprinsäure zu einer im Handel erhältlichen Milch zugegeben. Wie man **Tabelle 2** entnehmen kann, werden befriedigende Ermittlungsdaten erzielt (95 bis 101 %). **Figur 6** zeigt die Beziehung zwischen der Lagerzeit gerührter Rohmilch bei 25 °C und der Sensorantwort. Die Sensorantwort erhöht sich mit der Lagerzeit der Rohmilch, was nahelegt, daß das erfindungsgemäße System zur Aufzeichnung der Lipolyse von Rohmilch beim Lagern geeignet ist.

**Tabelle 2**

| Ermittlung von zu Milch zugegebenen Fettsäuren | | | | |
|---|---|---|---|---|
| Fettsäure | Zugabe | Gehalt | gef. | wiedergef. (%) |
| | Konzentration (mM) | | | |
| Buttersäure | 0 | 0.65 | 0.65 | - |
| | 0.28 | 0.93 | 0.88 | 95 |
| | 0.56 | 1.21 | 1.20 | 99 |
| | 1.12 | 1.77 | 1.69 | 95 |
| | 2.80 | 3.45 | 3.39 | 98 |
| Caprinsäure | 0 | 0.41 | 0.41 | - |
| | 0.29 | 0.70 | 0.71 | 101 |
| | 0.58 | 0.99 | 0.98 | 99 |
| | 1.45 | 1.86 | 1.86 | 100 |

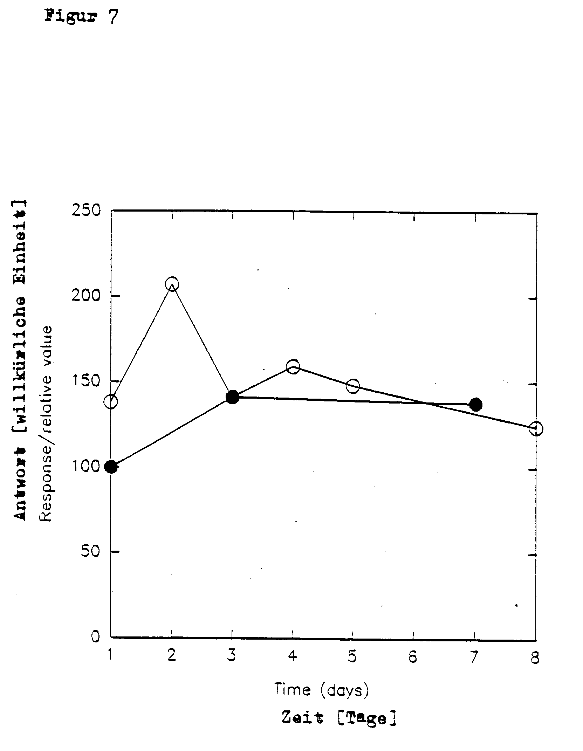

**Lagerstabilität und Betriebsstabilität**. Es wird die Lagerstabilität des immobilisierten Mikroorganismus und die Betriebsstabilität der erfindungsgemäßen mikrobiellen Elektrode untersucht. Dazu werden drei mikrobielle Elektroden unter identischen Bedingungen hergestellt (**Figur 7**). Die Antwort der ersten Elektrode wird am Tag der Herstellung bestimmt. Die restlichen Elektroden werden im Nährmedium mit einem Gehalt an Buttersäure bei 5 °C gelagert, bevor sie eingesetzt werden. Wenn die Antworten der zweiten und der dritten Elektrode am dritten Tage und am siebenten Tage nach der Herstellung bestimmt werden, stellt man keine Verminderung im Vergleich mit der Antwort am ersten Tage fest.

Die Betriebsstabilität der mikrobiellen Elektrode wird dadurch bestimmt, daß man die dritte Elektrode für die fortlaufende Bestimmung von 10 Proben im 5h-Betrieb an einem Tage verwendet. Wie man **Figur 7** entnehmen kann, läßt sich keine signifikante Abnahme der Antwort selbst nach acht Tagen feststellen. Diese Ergebnisse zeigen, daß die erfindungsgemäße mikrobielle Elektrode eine gute Stabilität selbst noch zwei Wochen nach der Herstellung besitzt.

Nachstehend wird die Erfindung anhand der Figuren und experimenteller Daten noch näher erläutert.

Figur 1: Schematisches Diagramm eines Fließsystems; C = Trägerlösung; P = Pumpe; S = Probenlösung; MC = Mischschlange; FC = Durchflußzelle mit mikrobieller Elektrode; W = Abfall.

Figur 2: pH-Abhängigkeit einer mikrobiellen Elektrode; Buttersäurekonzentration 1,68 mM; Strömungsrate 1,0 ml/min.

Figur 3: Einfluß der Fließrate auf die Antwort (●) und Zeit zur Basislinienumkehr (o); Buttersäurekonzentration 1,68 mM.

Figur 4: Typischer Verlauf einer Antwort für Buttersäure; a = 0,11 mM; b = 0,28 mM; c = 0,56 mM; d = 0,84 mM; e = 1,12 mM; f = 1,68 mM; g = 2,24 mM; h = 2,80 mM; i = 3,36 mM.

Figur 5: Einfluß der Zellmenge in der PVA-Membran auf die Antwort; die Bedingungen entsprachen denen von Figur 2.

Figur 6: Beziehung zwischen der Lagerzeit gerührter Rohmilch bei 25 °C und der Sensorantwort; die Sensorantworten sind als entsprechende Buttersäurekonzentration dargestellt.

Figur 7: Lagerstabilität eines immobilisierten Mikroorganismus (●) und Betriebsstabilität einer mikrobiellen Elektrode (o).

**Materialien**. Kasein (Natriumsalz aus Rindermilch) und Dialysemembran (Zelluloserohr von Sigma Chemical Co.; das Zelluloserohr würde 90 bis 99 % Cytochrom C vom MW 12400 einer Cytochrom-C-Lösung über 10 h zurückhalten). Polyvinylalkohol (PVA vom MW etwa 22000) von Serva Feinchemica Co. Die anderen Reagenzien wurden mit Analysenqualität verwendet. Bei allen Arbeiten wurde destilliertes Wasser eingesetzt.

**Kultur und Immobilisierung des Mikroorganismus**. Arthrobacter nicotiana wurde unter aeroben Bedingungen bei 30 °C 18 h lang in einer 100-ml-Flasche kultiviert, die 25 ml eines Mediums enthielt, das sich aus 0,3 % Hefeextrakt, 0,5 % Pepton, 0,5 % Dikaliumhydrogenphosphat, 0,5 % Kadiumdihydrogenphosphat (jeweils Gewicht/Volumen) und 1 % Buttersäure (Volumen/Volumen) zusammensetzte. Das Medium wurde bei 4800 U/min 10 min lang zentrifugiert und die abzentrifugierten Zellen wurden mit etwa 100 ml einer 0,9-proz. Natriumchloridlösung gewaschen. Eine vorgegebene Menge der Zellen wurde mit Kaliumphosphatpuffer (0,1 M; pH 7,0) mit einem Gehalt an 5 % PVA gemischt. Die Zellsuspension wurde auf eine Glasplatte (5 cm x 2 cm) ausgebreitet und bei Raumtemperatur 3 h lang und danach über Nacht bei 5 °C getrocknet.

**Aufbau einer mikrobiellen Elektrode**. Die eingesetzte mikrobielle Elektrode wies eine Mikroorganismusmembran, eine Teflonmembran, eine Dialysemembran und eine Sauerstoffelektrode (Schott Geräte GmbH) auf. Die PVA-Membran mit dem immobilisierten Mikroorganismus wurde scheibenförmig ausgeschnitten (5,9 mm Durchmesser) und über der Teflonmembran der Sauerstoffelektrode befestigt. Die Dialysemembran wurde als Schutzmembran eingesetzt, um die Mikroorganismusmembran auf der Elektrode abzudecken und zu fixieren.

Es wurden freie Fettsäuren in der Pufferlösung durch Einsatz von Kasein emulgiert; dabei wurde ein Kaliumphosphatpuffer (0,1 M) mit einem Gehalt an 3 % Kasein (Gewicht/Volumen) als Reaktionspuffer verwendet. Freie Fettsäuren wurden zu dieser Lösung zugegeben und durch Ultraschallbehandlung (10 min) emulgiert; die auf diese Weise gebildete Emulsion diente als Probelösung. Die Emulsion war einen Tag lang stabil.

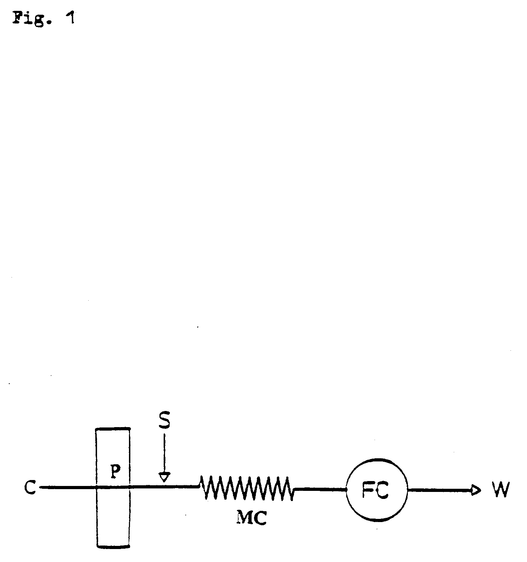

**FIA-System**. Ein schematisches Diagramm des Fließsystems ist in **Figur 1** gezeigt. Kaliumphosphatpuffer (0,1 M, pH 7,0) diente als Trägerlösung. Die Trägerlösung mit injizierter Probenlösung (35,5 µl) wurde durch eine Mischschlange (0,8 mm innerer Durchmesser und 79 cm Länge) gepumpt und zu einer Durchflußzelle transportiert, die mit der mikrobiellen Elektrode ausgerüstet war. Als die Fließrate auf 1,0 ml/min eingestellt wurde, betrug die Dispersion der injizierten Probenlösung etwa 8. Die Messungen wurden bei 30 °C ausgeführt, wobei die Peakgröße als Antwort aufgezeichnet wurde.

### Literatur

1 P. Walstra und R. Jenness, Dairy Chemistry und Physics, Wiley, New York, 1984, p. 336.
2 S. Kuzdzal-Sovoie, Bull Int. Dairy Fed., 118, 53 (1980)
3 V.P. Dole und H. Meinertz, J. Biol. Chem., 235, 2595 (1960).
4 K. Itaya und M. Ui, J. Lipid Res., 6, 16 (1965).
5 C. de Jong und H. T. Badings, J. High Resolution Chromotogr., 13, 94 (1990).
6 H. C. Deeth, C. H. Fitz-Gerald und A. J. Snow, N. Z. J. Dairy Sci. Technol., 18, 13 (1983).
7 S. Shimizu und H. Yamada in H. U. Bergmeyer (Eds.), Methods in Enzymatic Analysis, VCH Verlagsgesellschaft, Weinheim, 1985, vol. 8, p. 19.
8 K. Hosaka, T. Kikuchi, N. Mitsuhida und K. Kawaguchi, J. Biochem., 89, 1799 (1981).
9 K. Sode, E. Tamiya, I. Karube und Y. Kameda, Anal. Chim. Acta, 220, 251 (1989).
10 G. Wagner und R. D. Schmid,
11 M. Sugiura, T. Oikawa, K. Hirano und T. Inukai, Biochem. Biophys. Acta, 488, 353 (1977)
12 Fitz-Gerald, C. H.; Deeth, H. C.; Kitchen, B. J. (1981) The relationship between the levels of free fatty acids, lipoprotein lipase, carboxylesterase, N-acetyl-β-D-glucosaminidase, somatic cell count und other mastitis indices in bovine milk. J. Dairy Res., 48, 253-265.
13 Krisch, K. (1971) in "The Enzymes" ed. by P. D. Boyer; Academic Press: New York, vol. 5, pp 43-69.

## Patentansprüche

1. Arthrobacter-nicotiana-Stamm mit Acyl-CoA-Oxidase-Aktivität, die für C₄₋₁₂-Fettsäuren spezifisch ist.

2. Arthrobacter-nicotiana-Stamm DSM 6707.

3. Acyl-CoA-Oxidase-Aktivität, die für C₄₋₁₂-Fettsäuren spezifisch ist, isolierbar aus einem Arthrobacter-nicotiana-Stamm gemäß Anspruch 1 oder 2.

4. Verfahren zur Isolierung eines Arthrobacter-nicotiana-Stamms gemäß Anspruch 1 oder 2 oder zur Isolierung einer Acyl-CoA-Oxidase-Aktivität gemäß Anspruch 3, dadurch ***gekennzeichnet***, daß man
- mit Hilfe eines Sensors, der eine Sauerstoffelektrode und einen beliebigen Arthrobacter-nicotiana-Stamm umfaßt, der auf einem Träger immobilisiert ist, in Gegenwart eines wässrigen Mediums mit einem Gehalt an C₄₋₁₈-Fettsäuren testet, ob der Stamm eine Acyl-CoA-Oxidase-Aktivität besitzt, die für C₄₋₁₂-Fettsäuren spezifisch ist, und
- einen Stamm, für den man eine derartige Aktivität ermittelt, isoliert.

5. Sensor zur Ermittlung von Fettsäuren, umfassend eine Sauerstoffelektrode und eine Acyl-CoA-Oxidase-Aktivität, ***gekennzeichnet*** durch eine Acyl-CoA-Oxidase-Aktivität, die für C₄₋₁₂-Fettsäuren spezifisch ist.

6. Sensor nach Anspruch 5 , dadurch ***gekennzeichnet***, daß er ferner eine Acyl-CoA-Synthetase-Aktivität umfaßt.

7. Sensor nach Anspruch 5 oder 6, dadurch ***gekennzeichnet*****,** daß die Acyl-CoA-Oxidase-Aktivität und/oder die Acyl-CoA-Synthetase-Aktivität immobilisiert ist.

8. Sensor nach einem der Ansprüche 5 bis 7, ***gekennzeichnet*** durch eine Acyl-CoA-Oxidase-Aktivität in Form eines Arthrobacter-nicotiana-Stamms gemäß Anspruch 1 oder 2.

9. Sensor nach Anspruch 8, dadurch ***gekennzeichnet**,* daß der Arthrobacter-nicotiana-Stamm mit Hilfe von Polyvinylalkohol, vorzugsweise auf einem Polyvinylalkoholträger immobilisiert ist.

10. Sensor nach einem der Ansprüche 5 bis 9, dadurch ***gekennzeichnet***, daß der Sensor eine Schutzmembran trägt, die die Acyl-CoA-Oxidase-Aktivität schützt.

11. Sensor nach Anspruch 10, ***gekennzeichnet*** durch eine Schutzmembran aus Zellulose.

12. Sensor nach Anspruch 10 oder 11, ***gekennzeichnet*** durch eine Zellulose-Schutzmembran einer Trenngrenze von etwa 12 000.

13. Verwendung des Sensors gemäß einem der Ansprüche 5 bis 12 zur Bestimmung des Gehalts an C₄₋₁₂-Fettsäuren in flüssigen Medien, insbesondere in Molkereiprodukten.

14. Verwendung des Sensors gemäß einem der Ansprüche 5 bis 12 zur Mastitis-Bestimmung.

15. Verwendung nach Anspruch 13 oder 14 bei der Fließ-Injektions-Analyse (FIA).

16. Verwendung nach Anspruch 13 oder 14, wobei das System, in dem die FIA durchgeführt wird, die Zufuhr (C) einer Trägerlösung, die Zufuhr (S) der Probenlösung, eine Pumpe (P), eine Mischzone (MC), beispielsweise eine Mischschlange, eine Durchflußzelle (FC) mit einem Sensor gemaß einem der Ansprüche 5 bis 12, und eine Abfuhr (W) vorsieht.
